(19) **Europäisches Patentamt**
**European Patent Office**
**Office européen des brevets**

(11) **EP 1 553 979 B1**

(12) **EUROPEAN PATENT SPECIFICATION**

(45) Date of publication and mention
of the grant of the patent:
**22.08.2007 Bulletin 2007/34**

(51) Int Cl.:
*A61K 39/295* (2006.01)    *A61K 39/205* (2006.01)
*A61K 39/12* (2006.01)    *A61K 39/145* (2006.01)
*C12N 15/85* (2006.01)    *C12N 15/62* (2006.01)

(21) Application number: **03769280.3**

(22) Date of filing: **16.09.2003**

(86) International application number:
**PCT/EP2003/010305**

(87) International publication number:
**WO 2004/026338 (01.04.2004 Gazette 2004/14)**

(54) **IHNV G FUSION PROTEIN FOR IMMUNE STIMULATION**

IHNV G FUSIONSPROTEIN FÜR DIE IMMUNSTIMULATION

PROTEINE DE FUSION COMPRENANT LA PROTEINE G D'IHNV POUR LA STIMULATION
IMMUNITAIRE

(84) Designated Contracting States:
**AT BE BG CH CY CZ DE DK EE ES FI FR GB GR
HU IE IT LI LU MC NL PT RO SE SI SK TR**

(30) Priority: **17.09.2002 GB 0221552**
**17.09.2002 GB 0221553**

(43) Date of publication of application:
**20.07.2005 Bulletin 2005/29**

(73) Proprietors:
• **Novartis AG**
**4056 Basel (CH)**
Designated Contracting States:
**BE BG CH CY CZ DE DK EE ES FI FR GB GR HU
IE IT LI LU MC NL PT RO SE SI SK TR**
• **Novartis Pharma GmbH**
**1235 Vienna (AT)**
Designated Contracting States:
**AT**

(72) Inventors:
• **Simard, Nathalie C.**
**Federicton,**
**New Brunswick E3B 7G2 (CA)**
• **Bootland, Linda M.**
**Crapaud,**
**Prince Edward COA 1JO (CA)**

(74) Representative: **Ott, Johann**
**Corporate Intellectual Property,**
**Novartis AG**
**4002 Basel (CH)**

(56) References cited:
**WO-A-03/097090**

• **DESMEZIERES E ET AL: "Lyssavirus
glycoproteins expressing immunologically
potent foreign B cell d cytotoxic T lymphocyte
epitopes as prototypes for multivalent ccines"
JOURNAL OF GENERAL VIROLOGY, SOCIETY
FOR GENERAL MICROBIOLOGY, READING, GB,
vol. 80, no. PART 9, September 1999 (1999-09),
pages 2343-2351, XP002145723 ISSN: 0022-1317**
• **LORENZEN N ET AL: "DNA vaccines as a tool for
analysing the protective immune response
against rhabdoviruses in rainbow trout" FISH
AND SHELLFISH IMMUNOLOGY, vol. 12, no. 5,
May 2002 (2002-05), pages 439-453, XP002269214
ISSN: 1050-4648**
• **BOUDINOT P ET AL: "Combined DNA
Immunization with the Glycoprotein Gene of Viral
Hemorrhagic Septicemia Virus and Infectious
Hematopoietic Necrosis Virus Induces Double-
Specific Protective Immunity and Nonspecific
Response in Rainbow Trout" VIROLOGY,
ACADEMIC PRESS,ORLANDO, US, vol. 249, no.
2, 30 September 1998 (1998-09-30), pages
297-306, XP004445647 ISSN: 0042-6822**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 553 979 B1

- NOONAN ET AL: "Recombinant infectious hematopoietic necrosis virus and viral hemorrhagic septicemia virus glycoprotein epitopes expressed in Aeromonas salmonicida induce protective immunity in rainbow trout (Oncorhynchus mykiss)" APPLIED AND ENVIRONMENTAL MICROBIOLOGY, WASHINGTON,DC, US, vol. 61, no. 10, 1 October 1995 (1995-10-01), pages 3586-3591, XP002087051 ISSN: 0099-2240

- BIACCHESI STEPHANE ET AL: "Heterologous exchanges of the glycoprotein and the matrix protein in a Novirhabdovirus" JOURNAL OF VIROLOGY, vol. 76, no. 6, March 2002 (2002-03), pages 2881-2889, XP002269215 ISSN: 0022-538X

SEQUENCE LISTING

<110> Novartis AG

<120> IHNV G Protein for Immune Stimulation

<130> 32647

<140> PCT/EP2003/10305
<141> 2003-09-16

<150> GB 0221552.3
<151> 2002-09-16

<150> GB 0221553.1
<151> 2002-09-16

<160> 1

<170> PatentIn version 3.3

<210> 1
<211> 20
<212> PRT
<213> Infectious hematopoietic necrosis virus

<400> 1

```
Met Asp Thr Met Ile Thr Thr Pro Leu Ile Leu Ile Leu Ile Thr Cys
1               5                   10                  15

Gly Ala Asn Ser
                20
```

**Description**

Field of the Invention

The present invention relates to use of a novel class of vaccines for preventing infectious diseases, particularly in fish. The invention also relates to a vaccine comprising a portion of the nucleic acid sequence encoding the G protein of IHNV (Infectious Haematopoietic Necrosis Virus), and a portion of the nucleic acid sequence encoding a second peptide, the second peptide being derived from a pathogen.

Background of the Invention

Recombinant vaccines are occasionally employed in human and veterinary medicine as an alternative to more traditional approaches based on killed or attenuated pathogens. Many foreign antigens delivered systemically to the body in this way are capable of activating only one arm of the immune system, by stimulating the humoral immune response to generate antibodies by the Major Histocompatibility Complex (MHC) class II pathway. However, an ideal vaccine should also induce a cellular response by destruction of infected cells through activation of the MHC class I pathway. The latter response is achieved through cytosolic degradation of foreign protein in infected cells, such that fragments of the foreign material are associated with MHC class I molecules and shuttled to the cell surface for presentation to CD8[+] cytotoxic T cells (CTL).

Nucleic acid vaccines (NAVs) are a relatively new form of technology which are useful for delivery of pathogen antigens, especially viral antigens. As the viral proteins encoded by the vaccines are expressed *in situ* by the host's cellular apparatus, theory suggests that they should elicit a cell-mediated immune response capable of protecting animals when challenged. Results, however, have been mixed: in fish, NAVs expressing the infectious haematopoietic necrosis virus (IHNV) G protein (surface glycoprotein), and the viral haemorrhagic septicaemia virus G protein are effective against IHNV and VHSV infections, respectively. However, it has been difficult to demonstrate convincing protection of fish using NAVs based on other viral antigens.

For example, Boudinot et al. (1998), Virology, 249, 297-306 were double-immunised by coexpression of G proteins of IHNV and VHSV on separate plasmids. The kinetics and intensity of the response to the combination of G proteins were similar to those induced by expression of a single G protein.

It is an object of the present invention to provide improved vaccines effective against a variety of diseases in fish and other animals caused by infection with pathogens.

Summary of the Invention

In a first aspect, the invention provides an expression vector comprising a portion of the IHNV G protein coding nucleic acid sequence, fused in frame with a portion of a second protein coding sequence from a pathogenic organism other than IHNV. The portion of the IHNV G protein is optionally the isolated leader sequence of IHNV G protein. The second protein is preferably an antigen from a pathogen of fish, optionally a virus. The portion of the IHNV G protein coding sequence is fused with the second protein coding sequence.

The invention also provides a vaccine composition comprising a first expression vector carrying a portion of the IHNV G protein coding nucleic acid sequence fused in frame with a portion of the coding nucleic acid sequence of a second antigen other than an antigen of IHNV carried on said first expression vector together with a pharmaceutically acceptable carrier. In one embodiment, the vaccine composition comprises an adjuvant.

In another aspect, the invention provides use of a composition in the manufacture of a medicament for the prevention or treatment of an infectious disease in an animal, wherein said composition comprises a first expression vector comprising a portion of the IHNV G protein coding sequence, fused in frame with a portion of a second protein coding sequence from a pathogenic organism responsible for said infectious disease, wherein IHNV is not a causative agent of the infectious disease.

In a further aspect, the invention provides use of a composition in the manufacture of a medicament for the prevention or treatment of an infectious disease in fish other than IHNV, wherein said composition comprises a portion of the IHNV G protein coding sequence, and further comprises a portion of a second protein coding sequence from a pathogenic organism responsible for said infectious disease carried on the same expression vector.

Detailed description of the Invention

The invention is based on the observation that the presence of IHNV G protein or its leader sequence (also termed signal sequence) expressed from a nucleic acid vaccine in frame with a second antigen can boost the immune response, thereby enhancing protection against the pathogen from which the second antigen is derived. The explanation for this effect has not yet been elucidated, but it may relate to the existence of immunostimulatory motifs on the G protein. It is also plausible that the fusion of the antigenic protein to the G protein or its leader sequence results in the translocation of the antigen to the cell surface, thus increasing the exposure of the peptide to the host's immune system. Alternatively, an increase in protection may be due to synergy through a combination of these effects.

IHNV G protein has been used in recombinant form as the basis for vaccination of fish against IHNV (US 5,534,555). The amino acid and nucleic acid sequences of the G protein are known, for instance, from Koener et al. (1987) J. Virol. 61: 1342-1349, which is incorporated herein by reference.

IHNV is one member of the family of rhabdoviruses, which share the "G" glycoprotein. Rhabdoviruses include the vesiculoviruses (e.g. Vesicular stomatitis virus (VSV)), the lyssaviruses (e.g. rabies virus), the ephemoviruses (e.g. bovine ephemeral fever virus) and the novirhabdoviruses.

The immune-boosting effect of rhabdovirus G protein sequences can be applied to treatment or prevention of disease in any animal having both humoral and cellular branches to the immune system. Such animals include mammals of all varieties (including humans), fish, birds, and reptiles.

We report on one experiment (Example 1) in which the IHNV G protein and the VP2 antigen from IPNV (Infectious Pancreatic Necrosis Virus) are expressed together as a fusion protein on a single DNA plasmid *in vivo* in fish. Recombinant IPNV VP2 has previously been expressed in organisms such as *E. coli,* and used for vaccination of fish against IPNV, with a certain degree of success. US 5,165,925 relates to a vaccine against IPNV comprising the VP2 polypeptide.

This combination nucleic acid vaccine (NAV) was injected into fish, which were subsequently challenged with IPNV, as described in Example 1. We observed a marked improvement in survival when compared with immunization using a conventional viral preparation, namely an oil-adjuvanted killed virus. In fact, the relative percentage survival (RPS) with the fusion G protein-VP2 protein NAV was over 50% when compared to the PBS negative control, while the killed virus had an RPS of about 25%.

The construct carrying the IPNV VP2 protein without the IHNV G protein resulted in a mean RPS of just 31% relative to the PBS negative control. Therefore, inclusion of the IHNV G protein in the construct has the effect of boosting the immune response to the IPNV VP2 protein to generate a level of protection 67% stronger than with the VP2 protein alone.

The immune-stimulating effect of the IHNV G protein was verified in a second experiment (Example 2) in which the leader sequence of IHNV G protein was fused 5' of the ISAV hemagglutinin (HA) gene on a DNA expression vector and the vector was used to vaccinate fish against ISAV infection in a challenge trial. The presence of the IHNV G protein leader sequence significantly boosts the protective effect of the ISAV HA antigen in a DNA vaccine.

The invention encompasses both nucleic acid vaccines and vaccines based on recombinant antigens. A recombinant antigen vaccine comprises isolated or purified IHNV G protein (or a portion thereof) and an isolated or purified portion of a second antigen from a fish pathogen other than IHNV. The portion of the IHNV G protein and the portion of the second antigen are provided together in the form of a fusion protein.

An "isolated" or "purified" protein is defined as being substantially free of cellular material or other contaminating proteins from the cell or tissue source from which the protein is derived, or substantially free from chemical precursors or other chemicals when chemically synthesized.

A preferred vaccine is a nucleic acid vaccine carrying a portion of the IHNV G protein gene fused in frame with a portion of a second gene from a pathogen other than IHNV on the same DNA expression vector. The nucleic acid sequence encoding the portion of the G protein, or a fragment thereof, and the second antigen, or a fragment thereof, are fused in frame.

When genes are fused in frame it is meant that the register of the triplet code in the nucleic acid sequences recognized by tRNA molecules is identical to the register in the naturally-occurring genes, so that the resulting translated amino acid sequence is a peptide comprising a portion of the IHNV G protein and a portion of the second peptide. Preferably, these two genes or gene fragments are fused directly, without any intervening sequence. When the IHNV G protein and second protein gene sequences or portions thereof are fused in tandem, the IHNV G protein sequence or portion thereof can be 5' of the second protein sequence, 3' of the second protein sequence, or embedded within that sequence.

For present purposes a "portion" of a protein is understood to mean any peptide molecule having at least 7, optionally at least 15, or at least 25, or at least 50, or at least 100 contiguous amino acids of the reference protein. A "portion" of a gene or nucleic acid sequence is any part of that gene sequence comprising at least 20, optionally at least 50, or at least 100, or at least 200 consecutive nucleotides of the complete coding sequence. A "portion" of a gene or protein may be the full-length gene sequence or amino acid sequence. In a preferred embodiment the portion of the IHNV G protein used in the invention comprises the outer membrane-targeting leader sequence of the G protein, or its encoding nucleotide sequence. Optionally, this portion comprises the leader sequence to the exclusion of the rest of the G protein. The complete leader sequence (reading from the N-terminus) is: MDTMITTPLILILITCGANS (SEQ ID NO:1). A truncated but functional version of the leader sequence may be employed in place of the complete leader sequence.

When reference is made to the IHNV G protein or the second protein, or their respective coding sequences, it should be understood that this term incorporate proteins or encoding sequences with substantial homology. "Substantially homologous" in this context means that a sequence, when compared to a reference sequence, has at least 60% homology, preferably at least 70% homology, more preferably at least 80% homology, more preferably at least 90% homology, and most preferably at least 95% homology to the reference sequence.

To determine the percent homology of two amino acid sequences or of two nucleic acid sequences, the sequences are aligned for optimal comparison purposes (e.g. gaps can be introduced in the sequence of a first amino acid or nucleic acid sequence for optimal alignment with a second amino acid or nucleic acid sequence and the intervening non-homologous sequence in the gap can be disregarded for comparison purposes).

When a position in the first (reference) sequence is occupied by the same amino acid residue or nucleotide as the corresponding position in the sequence, the molecules are homologous at that position (i.e. there is identity at that position). In the case of nucleic acid sequence comparison there is also homology at a certain position where the codon triplet including the nucleotide encodes the same amino acid in both molecules being compared, due to degeneracy of the genetic code.

The percent homology between two sequences is a function of the number of homologous positions shared by the sequences (i.e., % homology = no. of homologous positions/total no. of positions). Optionally, the comparison of sequences and determination of percent homology can be accomplished using a mathematical algorithm. Suitable algorithms are incorporated in the NBLAST and XBLAST programs of Altschul et al. (1990) J. Mol. Biol. 215:430-10.

Furthermore, amino acids with identical charges may be substituted for each other. For example, lysine and arginine may be substituted for each other. Glutamic acid and aspartic acid can be substituted for each other. Glutamate and aspartate can also be substituted for each other. Such charge neutral changes to amino acids of a protein are recognized in the field and the resulting protein would still be covered by this invention.

There are many different geographical isolates of IHNV and other pathogens. There is a certain degree of variation in nucleic acid sequence of these pathogens and in the amino acid sequences of the proteins they expressed. The IHNV G protein and second protein used in the invention are not restricted to any specific isolate source. There may be an advantage in matching the second protein variant with the prevalent isolates in a particular geographical zone when designing a vaccine for that area.

In one embodiment of the invention there is provided a DNA expression vector in which nucleic acid sequences for IHNV G protein and a second protein are operably linked to transcriptional regulatory sequences, and a nucleic acid vaccine comprising the DNA expression vector and a pharmaceutically acceptable carrier. The nucleic acid sequences for the IHNV G protein and the second protein may be linked in order to be expressed under the control of the same transcriptional regulatory sequence(s), or they may be expressed independently from one another under the control of separate transcriptional regulatory sequences. As used herein, the term "DNA expression vector" refers to a nucleic acid molecule capable of transporting another nucleic acid to which it has been linked. Preferably the DNA expression vector is a eukaryotic expression vector. One type of vector is a "plasmid", which refers to a circular double stranded DNA loop into which additional DNA segments can be ligated. Another type of vector is a viral vector, wherein additional DNA segments can be ligated into the viral genome. Within a recombinant expression vector, "operably linked" is intended to mean that the nucleotide sequence of interest is linked to the regulatory sequence(s) in a manner which allows for expression of the nucleotide sequence.

Transcriptional regulatory sequences include promoters and polyadenylation sequences. The immune response can be enhanced using other nucleotide sequences such as immune-stimulating oligonucleotides having unmethylated CpG dinucleotides, or nucleotide sequences that code for other antigenic proteins or adjuvanting cytokines. Regulatory sequences include those which direct constitutive or inducible expression of a nucleotide sequence in many types of host cell and those which direct expression of the nucleotide sequence only in certain host cells (e.g., tissue-specific regulatory sequences). The DNA can be present in naked form or it can be administered together with an agent facilitating cellular uptake (e.g. liposomes or cationic lipids). The technology of DNA vaccination is reviewed for instance in WO 90/11092, incorporated herein by reference.

For optimal in vivo expression in fish it may be preferred to select transcriptional regulatory sequences endogenous to the fish to be vaccinated. For instance, endogenous cytokine or actin gene promoters may be considered, or other regulatory sequences may be derived from fish DNA viruses. DNA vaccination as applied to fish is explained in more detail in US 5,780,448.

Recombinant IHNV and other pathogen proteins have been successfully expressed in a variety of organisms, including *E. coli* and *Pichia pastoris,* using vectors containing constitutive or inducible promoters. In a recombinant vaccine purified IHNV G protein and second protein may be mixed together for co-administration. Alternatively, an expression vector comprising a fusion of portions of these two genes can be constructed by standard techniques and expressed within a host cell, in order to prepare a purified recombinant fusion protein. Conventional methods of protein purification can be employed to prepare the recombinant protein for use in a vaccine. Optionally, a lysate of host cells expressing recombinant protein may be used in place of recombinant protein which has undergone further purification procedures.

On the basis of the results demonstrated herewith, we describe a method of treating or preventing infectious disease in an animal such as a fish, comprising administering to the animal a composition comprising a portion of the nucleotide sequence of the G protein of IHNV (or the encoded protein sequence), and a portion of the nucleotide sequence of a second antigen from the pathogen causing the infectious disease (or the encoded protein sequence). We also claim the use of a composition comprising a portion of the G protein of IHNV, or its encoding nucleotide sequence, and a portion of a second protein from a pathogenic organism, or its encoding nucleotide sequence, in the manufacture of a medicament (vaccine) for the treatment or prevention of an infectious disease in an animal (e.g. a fish) caused by said pathogenic organism, and/or for the treatment or prevention of infection with said pathogenic organism.

The "second" (heterologous) protein of the invention can be a protein (or peptide or antigen) from a fish pathogen other than IHNV. The second protein may be from a fungal, viral, protozoan or bacterial fish pathogen which causes infectious disease syndromes. The second protein is optionally from a virus other than a rhabdovirus, or other than VHSV. For instance, the second protein may be derived from Infectious Salmon Anaemia Virus (ISAV), Infectious Pancreatic Necrosis Virus (IPNV), Iridovirus, Nervous Necrosis Virus (NNV), Salmon Pancreas Disease Virus (SPDV), Spring Viremia of Carp Virus (SVCV), Viral Hemorrhagic Septicemia Virus (VHSV), *Renibacterium salmoninarum* (causative agent of Bacterial Kidney Disease), *Piscirickettsia salmonis* (causative agent of Salmonid Rickettsial Septicemia), *Vibrio* spp, *Aeromonas* spp, *Yersinia ruckerii, Nocardia* spp., *Pseudomonas* spp., *Photobacterium damselae,* etc. In a preferred embodiment the second protein is of viral origin. A large and growing number of polypeptides from these and other pathogenic organisms have been purified and/or cloned and expressed and are available to be or provided in conjunction with IHNV G protein or its coding sequence in a vaccine composition. Preferred examples include IPNV proteins VP1, VP2, VP3 and NS and their coding nucleotide sequences; ISAV proteins disclosed in WO 01/10469 including hemagglutinin, nucleocapsid, polymerase and segment 7 P4 and P5 proteins, and their coding nucleotide sequences; *P. salmonis* proteins disclosed in WO 01/68865 including OspA and IcmE and their coding nucleotide sequences; nodavirus proteins such as the nucleocapsid; and structural polypeptides from SPDV and their coding nucleotide sequences (disclosed in WO 99/58639). A preferred vaccine composition according to the invention comprises a DNA expression vector carrying a portion of the IHNV G protein nucleotide sequence fused in-frame with a portion of the IPNV VP2 sequence or a portion of the IHNV G protein leader sequence fused in-frame with a portion of the ISAV hemagglutinin sequence.

The prime candidate fish species for receiving the vaccine of the invention are salmonid fish, including salmon and trout species, particularly coho salmon (*Oncorhynchus kisutch*), brook trout (*Salvelinus fontinalis*), brown trout (*Salmo trutta*), chinook salmon (*Oncorhynchus tshawytscha*), masu salmon (*Oncorhyncus masou*), pink salmon (*Oncorhynchus gorbuscha*), rainbow trout (*Oncorhynchus mykiss*)*,* Arctic charr (*Salvelinus alpinus*) and Atlantic salmon (*Salmo salar*). However, any other fish species susceptible to infectious disease may benefit, such as ornamental fish species, koi, goldfish, carp, catfish, yellowtail, sea bream, sea bass, pike, halibut, haddock, tilapia, turbot, wollfish, and so on.

The "second" (heterologous) protein of the invention can also be an antigen from a pathogen of animals other than fish, especially mammals such as humans. The second protein may be from a fungal, viral, protozoan or bacterial pathogen which causes infectious disease syndromes in animals. A non-limiting list of possible pathogens includes: hepatitis viruses (e.g. HBV, HCV), HIV and other immunodeficiency virus genes, influenza viruses, measles virus, coronaviruses, herpesviruses, poliovirus, rhinoviruses, rotaviruses, adenoviruses, papillomaviruses, hantaviruses, parvoviruses and the specific viruses Bovine Viral Diarrhea Virus (BVDV), Bovine Herpesvirus (BHV), Foot and mouth disease virus, Bovine Respiratory Syncytial Virus (BRSV), Parainfluenza type 3 virus (P13), Infectious Bovine Rhinotracheitis (IBR), Porcine Respiratory and Reproductive Syndrome Virus (PRRSV); species of *Giardia, Yersinia, Leishmania, Amoeba, Entamoeba, Trypanosoma, Toxoplasma, Plasmodium, Cryptosporidia, Candida, Cryptococcus, Histoplasma, Coccidioides, Blastomyces, Staphylococcus,* Streptococcus, *Pneumococcus, Neisseria, Listeria, Campylobacter, Chlamydia, Eimeria, Clostridia, Pasteurella, Brachyspira, Salmonella, Legionella, Mycobacteria, Mycoplasma* (e.g. *M. bovis, M. hyopneumoniae*), *Treponema, Borrelia, Leptospira, Ehrlichia, Rickettsia, Brucella, Neospora, Fusobacterium, E. coli, Mannheimia haemolytica, Haemophilus somnus, Actinobacillus pleuropneumoniae, Anaplasma,* etc.

Any vertebrate animal can be immunized with the vaccines of the invention. Particular mention can be made of humans, the major species of farmed land animals, namely cattle, horses, sheep, swine and poultry birds, and companion animals.

Two-component vaccines may be prepared together in a single vaccine composition, or they may be prepared separately for separate administration or for co-administration. Optionally, the individual components are provided in the form of a kit, for sequential, separate or simultaneous administration. The two components may be mixed together immediately prior to administration.

For fish, the preferred route of administration of the vaccines of the invention is by injection into the muscle (in particular, into the epaxial muscle). Alternative options are injection into the peritoneal cavity (for larger fish), orally in feed, or by immersion in sea water or in fresh water. It is recommended that fish be 2 grams or greater in body weight for administration of the vaccine of the invention by injection, preferably 10 grams or larger. For immersion or oral administration, it is preferred that fish have a body weight of at least 0.1g, optionally at least 0.5g, usually at least 2 grams.

In animals other than fish vaccines, in particular nucleic acid vaccines, are often delivered by intramuscular injection or by delivery to the mucosal membranes; delivery techniques are include, but are not limited to: electroporation, subcutaneous or transdermal injection, microinjection, jet injection, calcium phosphate-DNA co-precipitation, DEAE-dextran-mediated transfection, polybrene-mediated transfection, liposome fusion, lipofection, protoplast fusion, viral infection, microparticles, bacterial carriers, and biolistics (particle bombardment, e.g. using a gene gun).

The vaccine of the invention may be administered to animals for prophylactic or therapeutic purposes. The vaccine is capable of inducing long term protection against the target infectious disease. "Long term" protection in the case of fish means a protective immune response for longer than 7 days, more preferably longer than 20 days, and most preferably longer than 70 days post vaccination.

The effective dosage of vaccine may vary depending on the size and species of the subject, and according to the mode of administration. The optimal dosage can be determined through trial and error by a doctor, veterinarian or aquaculture specialist. For fish, vaccines may comprise between 0.01 and 0.5 g, preferably between about 0.05 and 0.2 g of recombinant protein in a single dosage. A suitable dosage range for nucleic acid vaccines may be as low as picogram, or as high as mg quantities, but is normally from about 0.01 to 100 $\mu$g, preferably 0.1 $\mu$g to 50 $\mu$g per unit dose, more preferably about 1 $\mu$g to 20 $\mu$g, and most preferably about 5 $\mu$g to 10 $\mu$g per unit dose. Due to the stress suffered by fish in response to vaccination, it is preferred that the vaccine is provided as a single shot vaccine, in single dosage form. For injectable vaccines, a single dosage unit is suitably 0.025 to 0.5 ml, preferably 0.05 to 0.2 ml, optionally about 0.1 ml, in volume.

Typically, vaccines are prepared as liquid solutions, emulsions or suspensions for injection or delivery in water. Solid (e.g. powder) forms suitable for dissolution in, or suspension in, liquid vehicles, or for mixing with solid food, prior to administration may also be prepared. The vaccine may be lyophilized, optionally freeze-dried, in a ready to use form for reconstitution with a sterile diluent. For instance, lyophilized vaccine may be reconstituted in 0.9% saline (optionally provided as part of the packaged vaccine product). Nucleic acid vaccines are particularly suited to lyophilisation due to the stability and long shelf-life of the molecules. Alternatively, the vaccine may be provided in a saline solution. Liquid or reconstituted forms of the vaccine may be diluted further in a small volume of water (e.g. 1 to 10 volumes) before addition to a pen, tank or bath for administration to fish by immersion. The pharmaceutical vaccine compositions of the invention may be administered in a form for immediate release or extended release.

Pharmaceutically acceptable carriers or vehicles include conventional excipients, and may be, for example, solvents such as water, oil or saline, dextrose, glycerol, sucrose, tricaine, wetting or emulsifying agents, bulking agents, coatings, binders, fillers, disintegrants, diluents, lubricants, pH buffering agents, or conventional adjuvants such as muramyl dipeptides, avridine, aluminium hydroxide, oils, saponins, block co-polymers and other substances known in the art. In the case of nucleic acid vaccines, the DNA expression vector may be delivered naked, or may be provided in the form of cationic lipid-DNA complexes, liposomes, calcium phosphate co-precipitates, adsorbed on microparticles, and so on.

In some instances it may be desirable to combine the vaccine of the invention with a conventional vaccine against an infectious pathogen (killed pathogen or recombinant pathogen antigen vaccine or pathogen nucleic acid vaccine) in a combination vaccine, or in a kit comprising both components for separate, sequential or simultaneous administration, for treatment or prevention of infectious disease caused by the pathogen.

Example 1

Evaluation of nucleic acid vaccines against Infectious Pancreatic Necrosis Virus in Atlantic salmon, *Salmo salar.*

Vaccination

Atlantic salmon parr (body weight 9-26g) are held in two 1 metre diameter circular tanks with freshwater at 8°C, and starved for 24 hours prior to vaccination. For vaccination, fish are anaesthetized in 3-aminobenzoic acid ethyl ester (MS222, Sigma, Poole, UK) at a concentration of approximately 0.5g/litre. Nucleic acid vaccines (10 $\mu$g DNA/50 $\mu$l dose) are administered by intramuscular injection on the left dorsal flank, in the area just below the dorsal fin. Oil adjuvanted killed IPNV vaccine, and PBS control are administered by intraperitoneal injection (100$\mu$l). Each treatment group has 40 fish, and there are 2 replicates per vaccine for each of 6 vaccines.

The test groups receive the following compositions:

    (1) the pUK vector: a cloning vector carrying the kanamycin resistance gene, the CMV immediate-early promoter, a multiple cloning site, and the bovine growth hormone polyadenylation signal (BGH polyA).

    (2) pUK +VP2: the pUK vector incorporating the entire coding sequence of IPNV VP2 within the vector multiple cloning site.

(3) pUK + IHNG: the pUK vector incorporating the entire coding sequence of IHNV G protein within the vector multiple cloning site.

(4) pUK + IHNG + VP2: the pUK vector incorporating the entire coding sequence of IPNV VP2 protein fused in-frame to the glycoprotein (G) of the IHN virus within the multiple cloning site, such that the G protein is 5' of the VP2.

(5) IPNV + oil: an inactivated preparation of IPN virus, adjuvanted with oil.

(6) PBS (negative control)

Nearly 6 weeks post-vaccination, the fish are smolted over a period of 5 days. The seawater flow into the fish tanks is gradually increased, while the freshwater flow is reduced, such that by the end of 5 days the fish are in full strength seawater.

Co-habitation Challenge

807 degree days after vaccination, a frozen pass 2 supernatant of IPN virus "Cole-Deep" strain is diluted five-fold in sterile PBS to give a final concentration of $1 \times 10^7$ $TCID_{50}$/ml. Fish are anaesthesised in MS222 and challenged in batches by intraperitoneal injection of 100 $\mu$l of virus suspension, such that each fish receives a dose of $10^6$ $TCID_{50}$. The seawater temperature at challenge is about 11°C, and the water flow rate is approximately 5 litres per minute in each tank.

Mortalities are removed twice daily on first observation. The trial is terminated 8 weeks after challenge.

Conclusions

| Vaccine | Mean mortality % | SD | Mean RPS relative to PBS |
|---|---|---|---|
| PBS | 42.6 | 10.4 | n/a |
| pUK | 39.7 | 10.4 | 6.90 |
| pUK + IHNG | 35.3 | 8.3 | 17.24 |
| pUK + VP2 | 29.4 | 4.2 | 31.03 |
| pUK + IHNG + VP2 | 20.6 | 4.2 | 51.72 |
| IPNV + oil | 31.7 | 5.1 | 25.65 |

The challenge model based on cohabitation with intraperitoneally injected Atlantic salmon smolts is successful: cumulative mortalities in intraperitoneally injected cohabitants reach an average of 41.25%, and this is closely replicated across the 4 challenge tanks with a standard deviation of 3.95%.

The performance of the killed IPNV vaccine given with oil is assessed in relation to the PBS vaccinated controls. The killed vaccine gives some protection, with a relative percent survival (RPS) compared to the PBS vaccinated controls of more than 25%.

The performance of the nucleic acid vaccines is compared to either the empty plasmid, or the plasmid containing the gene for the IHNV G protein. The control plasmids are compared to the sham vaccinated PBS controls to determine whether the empty plasmid or the plasmid containing the IHNV G protein has any protective effect. The plasmid vector pUK gives an insignificant protective effect. When the IPNV VP2 protein is included in the vector, significant protection (31% RPS) is given compared to PBS vaccinated controls. When the IHNV G protein gene is included in the vector, with no IPNV genes, the protective effect amounts to 17% RPS compared to PBS.

The vaccine with the most outstanding performance is the NAV containing the gene for IHNV G protein in tandem with the IPNV VP2. Fish vaccinated with this vaccine show a relative percent survival of 52% RPS compared to the PBS vaccinated controls.

In conclusion, the standard killed viral preparation with oil adjuvant performs adequately in this trial, but the performance of the NAVs containing VP2 is much greater. In particular, the NAV containing VP2 in tandem with IHNV G protein is most effective.

Example 2: Evaluation of nucleic acid vaccines against ISAV

Atlantic salmon parr of average weight 10g (< 6 months old) are acclimated for a minimum of 7 days to water at $12 \pm 1°C$ flowing at a rate of 2.5 Umin. The fish are fed a commercial pelleted diet at a daily rate of 1.5% body weight.

Prior to vaccination the fish are anaesthetized in 30mg/l benzocaine. Nucleic acid vaccines (10 μg DNA/50 μl dose) are administered by intramuscular injection into the epaxial muscle immediately anterior to the dorsal fin. Oil emulsion vaccine is administered by intraperitoneal injection (150μl). Each replicate vaccine group has 55 fish, and there are 2 replicates per vaccine for each of 5 vaccines.

The test groups receive the following compositions:

1) the pUK vector: a cloning vector carrying the kanamycin resistance gene, the CMV immediate-early promoter, a multiple cloning site, and the bovine growth hormone polyadenylation signal (BGH polyA).

(2) pUK-HA: the pUK vector incorporating the entire coding sequence of ISAV hemagglutinin protein in the multiple cloning site.

(3) pUK-IHNg: the pUK vector incorporating the entire sequence of the IHNV G protein in the multiple cloning site.

(4) pUK-HA-IHNg: the pUK vector incorporating the entire coding sequence of ISAV hemagglutinin protein in the multiple cloning site and fused at its 5' end to the IHNV G protein leader sequence.

(5) ISAV + oil: an inactivated preparation of ISA virus, adjuvanted with oil.

The fish are challenged at 850.5 degree days. A cohabitation challenge is used in which salmon of the same stock are adipose fin dipped, given an i.p. injection with 0.1ml cultured ISAV (about $10^4$ TCID$_{50}$ per fish), and added to each tank of treated fish.

Fish in each tank are monitored twice daily for mortality for 31 days. The relative percent survival (RPS) is calculated as follows:

$$RPS = 1 - (\% \text{ mortality of vaccines} / \% \text{ mortality of pUK control}) \times 100$$

Results:

The cumulative mortality for the negative control group (pUK plasmid) is 94%. The best protection is induced by injection of the positive control monovalent inactivated ISAV vaccine (94% RPS). Injection of the pUK-IHNg plasmid as a negative control induces some nonspecific protection (RPS 26%). pUK-HA confers a high level of protection (RPS 49%). This protection is significantly augmented by the addition of the IHNV G protein leader sequence at the 3' end (pUK-HA-IHNg) (RPS 60%). These results provide further support for the benefits of including the IHNV G protein sequence (or minimally, the leader sequence thereof) in a nucleic acid vaccine to boost immunity to an infectious disease other IHNV. The leader sequence of the G protein may be responsible for targeting the heterologous antigen to the cell surface within the fish, or there may be special motifs within the protein which non-specifically stimulate the fish's immune system.

| Test group | Mean mortality % | Standard error | RPS (relative to pUK) |
|---|---|---|---|
| pUK | 94 | 2 | - |
| ISAV-oil | 6 | 6 | 94 |
| pUK-HA | 48 | 4 | 49 |
| pUK-IHNg | 70 | 6 | 26 |
| pUK-HA-IHNg | 38 | 6 | 60 |

**Claims**

1. A DNA expression vector comprising a sequence encoding a portion of the infections haematopoïetic necrosis virus IHNV glycoprotein G protein coding sequence, and further comprising a portion of a second protein coding sequence from a pathogenic organism other than IHNV wherein the portion of the IHNV G protein coding sequence and the portion of the second protein coding sequence are fused in-frame on the same DNA expression vector.

2. A DNA expression vector according to claim 1 wherein said second protein coding sequence is from a fish pathogen.

3. A DNA expression vector according to any of claims 1 - 2 comprising the leader sequence of the IHNV G protein.

4. A DNA expression vector according to claim 3 wherein the leader sequence of the IHNV G protein is SEQ ID No.1.

5. A DNA expression vector according to any one of claims 1-4 comprising the complete IHNV G protein coding sequence.

6. A DNA expression vector according to any one of claims 1-5 wherein said second protein is from a pathogen selected from: ISAV, VHSV, IPNV, NNV, Iridovirus, SVCV, SPDV, Renibacterium salmoninarum, Piscirickettsia salmonis, Vibrio spp. , Aeromonas spp. , Yersinia ruckerii, Pseudomonas spp., Nocardia spp. and Photobacterium damselae subsp. piscicida.

7. A DNA expression vector according to claim 6 wherein the second protein is the VP2 protein from IPNV or the hemagglutinin protein from ISAV.

8. A nucleic acid vaccine comprising a DNA expression vector according to any one of claims 1-7 with a pharmaceutically acceptable carrier.

9. A fusion protein comprising a portion of the IHNV G protein and a portion of a second protein derived from a fish pathogenic organism other than IHNV.

10. A fusion protein according to claim 9 wherein said second protein is a pathogen selected from: ISAV, IPNV, NNV, Iridovirus, SVCV, SPDV, Renibacterium salmoninarum, Piscirickettsia salmonis, Vibrio spp. , Aeromonas spp. , Yersinia ruckerii, Pseudomonas spp., Nocardia spp. and Photobacterium damselae subsp. piscicida.

11. A fusion protein according to claim 10 wherein the second protein is VP2 from IPNV.

12. A fusion protein according to claim 11 wherein the second protein is hemagglutinin from ISAV.

13. A nucleic acid vaccine comprising a sequence encoding a fusion protein according to any one of claims 10-12 with a pharmaceutically acceptable carrier.

14. Use of a DNA expression vector according to any one of claims 1-7 or a fusion protein according to any one of claims 9-12 in the preparation of a medicament for the prevention or treatment of pathogenic infections in fish.

15. Use according to claim 14 in the preparation of a medicament for the prevention or treatment of bacterial, viral, fungal or protozoan infections in fish.

16. Use according to claim 14 or claim 15 in the preparation of a medicament for boosting the immune response thereby enhancing protection against the pathogen from which the second protein is derived.

**Patentansprüche**

1. DNA-Expressionsvektor, umfassend eine Sequenz, die einen Teil der Infectious Hematopoietic Necrosis Virus IHNV-Glycoprotein-G-Protein-codierenden Sequenz umfasst und weiterhin einen Teil einer zweiten Protein-codierenden Sequenz aus einem pathogenen Organismus, der anders ist als IHNV, umfasst, wobei der Teil der IHNV-G-Protein-codierenden Sequenz und der Teil der zweiten Protein-codierenden Sequenz im Raster auf dem gleichen DNA-Expressionsvektor fusioniert sind.

2. DNA-Expressionsvektor nach Anspruch 1, wobei die zweite Protein-codierende Sequenz aus einem Fischpathogen stammt.

3. DNA-Expressionsvektor nach einem der Ansprüche 1 bis 2, der die Leadersequenz des IHNV-G-Proteins einschließt.

4. DNA-Expressionsvektor nach Anspruch 3, wobei die Leadersequenz des IHNV-G-Proteins SEQ ID NO: 1 ist.

5. DNA-Expressionsvektor nach einem der Ansprüche 1 bis 4, der die vollständige IHNV-G-Protein-codierende Sequenz einschließt.

6. DNA-Expressionsvektor nach einem der Ansprüche 1 bis 5, wobei das zweite Protein aus einem Pathogen stammt, ausgewählt aus: ISAV, VHSV, IPNV, NNV, Iridovirus, SVCV, SPDV, Renibacterium salmoninarum, Piscirickettsia salmonis, Vibrio spp., Aeromonas spp., Yersinia ruckerii, Pseudomonas spp., Nocardia spp. und Photobacterium damselae subsp. piscicida.

7.  DNA-Expressionsvektor nach Anspruch 6, wobei das zweite Protein das VP2-Protein aus IPNV oder das Hämagglutininprotein aus ISAV ist.

8.  Nucleinsäureimpfstoff, der einen DNA-Expressionsvektor nach einem der Ansprüche 1 bis 7 mit einem pharmazeutisch verträglichen Träger einschließt.

9.  Fusionsprotein, das einen Teil des IHNV-G-Proteins und einen Teil eines zweiten Proteins, das aus einem Fisch-Pathogenorganismus, der anders ist als IHNV, stammt, einschließt.

10. Fusionsprotein nach Anspruch 9, wobei das zweite Protein ein Pathogen ist, ausgewählt aus ISAV, IPNV, NNV, Iridovirus, SVCV, SPDV, Renibacterium salmoninarum, Piscirickettsia salmonis, Vibrio spp., Aeromonas spp., Yersinia ruckerii, Pseudomonas spp., Nocardia spp. und Photobacterium damselae subsp. piscicida.

11. Fusionsprotein nach Anspruch 10, wobei das zweite Protein VP2 aus IPNV ist.

12. Fusionsprotein nach Anspruch 11, wobei das zweite Protein Hämagglutinin aus ISAV ist.

13. Nucleinsäureimpfstoff, der eine Sequenz, die ein Fusionsprotein nach einem der Ansprüche 10 bis 12 codiert, mit einem pharmazeutisch verträglichen Träger einschließt.

14. Verwendung eines DNA-Expressionsvektors nach einem der Ansprüche 1 bis 7 oder eines Fusionsproteins nach einem der Ansprüche 9 bis 12 bei der Herstellung eines Medikaments zur Prävention oder Behandlung von pathogenen Infektionen bei Fischen.

15. Verwendung nach Anspruch 14 bei der Herstellung eines Medikaments zur Prävention oder Behandlung von bakteriellen, viralen, fungalen oder protozoischen Infektionen bei Fischen.

16. Verwendung nach Anspruch 14 oder Anspruch 15 bei der Herstellung eines Medikaments zur Verstärkung der Immunantwort, um **dadurch** den Schutz gegen das Pathogen zu verbessern, aus dem das zweite Protein stammt.


**Revendications**

1.  Vecteur d'expression d'ADN comprenant une séquence codant pour une partie de la séquence codant pour la protéine G glycoprotéine du virus de la nécrose hématopoïétique infectieuse (IHNV), et comprenant en outre une partie d'une séquence codant pour seconde une protéine d'un organisme pathogène autre que l'IHNV, dans lequel la partie de la séquence codant pour la protéine G de l'IHNV et la partie de la séquence codant pour la seconde protéine sont fusionnées en phase sur le même vecteur d'expression d'ADN.

2.  Vecteur d'expression d'ADN selon la revendication 1, dans lequel ladite séquence codant pour la seconde protéine provient d'un agent pathogène des poissons.

3.  Vecteur d'expression d'ADN selon l'une quelconque des revendications 1 et 2, comprenant la séquence de tête de la protéine G de l'IHNV.

4.  Vecteur d'expression d'ADN selon la revendication 3, dans lequel la séquence de tête de la protéine G de l'IHNV est SEQ ID NO : 1.

5.  Vecteur d'expression d'ADN selon l'une quelconque des revendications 1 à 4, comprenant la totalité de la séquence codant pour la protéine G de l'IHNV.

6.  Vecteur d'expression d'ADN selon l'une quelconque des revendications 1 à 5, dans lequel ladite seconde protéine provient d'un agent pathogène choisi parmi : ISAV, VHSV, IPNV, NNV, Iridovirus, SVCV, SPDV, *Renibacterium salmoninarum, Piscirickettsia salmonis, Vibrio* spp., *Aeromonas* spp., *Yersinia ruckerii, Pseudomonas* spp., *Nocardia* spp. et *Photobacterium damselae* ssp. *piscicida.*

7.  Vecteur d'expression d'ADN selon la revendication 6, dans lequel la seconde protéine est la protéine VP2 de l'IPNV ou la protéine hémagglutinine de l'ISAV.

**8.** Vaccin à base d'acide nucléique comprenant un vecteur d'expression d'ADN selon l'une quelconque des revendications 1 à 7, avec un vecteur pharmaceutiquement acceptable.

**9.** Protéine de fusion comprenant une partie de la protéine G de l'IHNV et une partie d'une seconde protéine dérivée d'un organisme pathogène pour les poissons autre que l'IHNV.

**10.** Protéine de fusion selon la revendication 9, dans laquelle ladite seconde protéine est un agent pathogène choisi parmi : ISAV, IPNV, NNV, Iridovirus, SVCV, SPDV, *Renibacterium salmoninarum, Piscirickettsia salmonis, Vibrio* spp., *Aeromonas* spp., *Yersinia ruckerii, Pseudomonas* spp., *Nocardia* spp. et *Photobacterium damselae* ssp. *piscicida.*

**11.** Protéine de fusion selon la revendication 10, dans laquelle la seconde protéine est VP2 de l'IPNV.

**12.** Protéine de fusion selon la revendication 11, dans laquelle la seconde protéine est l'hémagglutinine de l'ISAV.^

**13.** Vaccin à base d'acide nucléique comprenant une séquence codant pour une protéine de fusion selon l'une quelconque des revendications 10 à 12, avec un vecteur pharmaceutiquement acceptable.

**14.** Utilisation d'un vecteur d'expression d'ADN selon l'une quelconque des revendications 1 à 7, ou d'une protéine de fusion selon l'une quelconque des revendications 9 à 12, dans la préparation d'un médicament destiné à la prévention ou au traitement d'infections pathogènes chez les poissons.

**15.** Utilisation selon la revendication 14, dans la préparation d'un médicament destiné à la prévention ou au traitement d'infections bactériennes, virales, fongiques ou protozoaires chez les poissons.

**16.** Utilisation selon la revendication 14 ou 15, dans la préparation d'un médicament destiné à stimuler la réponse immunitaire, améliorant ainsi la protection contre l'agent pathogène dont la seconde protéine est dérivée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5534555 A
- US 5165925 A
- WO 9011092 A
- US 5780448 A
- WO 0110469 A
- WO 0168865 A
- WO 9958639 A
- EP 200310305 W
- GB 0221552 A
- GB 0221553 A

**Non-patent literature cited in the description**

- **BOUDINOT et al.** *Virology,* 1998, vol. 249, 297-306
- **KOENER et al.** *J. Virol.,* 1987, vol. 61, 1342-1349
- **ALTSCHUL et al.** *J. Mol. Biol.,* 1990, vol. 215, 430-10